# EUROPÄISCHE PATENTSCHRIFT

(11) **EP 1 466 615 B1**
(45) Veröffentlichungstag und Bekanntmachung des Hinweises auf die Patenterteilung: **03.06.2009**
(21) Anmeldenummer: 04006076.6
(22) Anmeldetag: 09.09.1999
(51) Int. Cl.: A61K 38/12, A61P 7/00, A61P 9/00, A61P 19/00, A61P 27/00, A61P 31/00, A61P 35/00

(54) **Pharmazeutische Zubereitung**
Pharmaceutical composition
Composition pharmaceutique

(30) Priorität: 16.09.1998 DE 19842415
(43) Veröffentlichungstag der Anmeldung: 13.10.2004
(62) Teilanmeldung aus: 99969031.6
(73) Patentinhaber: Merck Patent GmbH, 64293 Darmstadt (DE)
(72) Erfinder: Jonczyk, Alfred, Dr., 64295 Darmstadt (DE); Perschl, Astrid, Dr., 64297 Darmstadt (DE); Goodman, Simon, Dr., 64347 Griesheim (DE); Roesner, Sigrid, Dr., 63110 Rodgau (DE); Haunschild, Jutta, Dr., 81739 Muenchen (DE)

(56) Entgegenhaltungen:
- WO-A-97/41844
- WO-A-97/45447
- WO-A-98/14192
- WO-A-98/31359
- DE-A- 19 534 177
- LODE H N ET AL: "Synergy between an antiangiogenic integrin alphav antagonist and an antibody-cytokine fusion protein eradicates spontaneous tumor metastases." PROCEEDINGS OF THE NATIONAL ACADEMY OF SCIENCES OF THE UNITED STATES OF AMERICA, 96 (4) 1591-6., 16. Februar 1999 (1999-02-16), XP002134006

## Beschreibung

Gegenstand der Erfindung ist die Verwendung von Cyclo-(Arg-Gly-Asp-D-Phe-NMe-Val) und/oder einem seiner physiologisch unbedenklichen Salze zur Herstellung eines Arzneimittels zur Bekämpfung von Tumoren bei der Radiotherapie.

Der Erfindung lag die Aufgabe zugrunde, verbesserte Behandlungsmethoden für Tumorerkrankungen zu ermöglichen.

Diese Aufgabe wurde durch den erfindungsgemäßen Gegenstand gelöst.

Cyclo-(Arg-Gly-Asp-D-Phe-NMe-Val) ist aus EP 0 770 622 bekannt und wirkt vor allem als Integrin-Inhibitor, wobei es insbesondere die Wechselwirkungen der α_{V}-, β₃- oder β₅-Integrin-Rezeptoren mit Liganden hemmt, wie z. B. die Bindung von Fibrinogen an den β₃-Integrinrezeptor. Besondere Wirksamkeit zeigt die Verbindung im Fall der Integrine α_{V}β₃, αvβ₅, α_{IIb}β₃ sowie α_{V}β1, αvβ₆ und α_{V}β₈.
Diese Wirkung kann z.B. nach der Methode nachgewiesen werden, die von J.W. Smith et al. in J. Biol. Chem. 265, 12267-12271 (1990) beschrieben wird.

Die vorliegende Erfindung ist in bezug auf EP 0 770 622 als Auswahlerfindung anzusehen.

In der WO 98/14192 sind pharmazeutische Präparate genannt, die Kombinationen von nicht-peptidischen Vitronektinrezeptorantagonisten mit Chemotherapeutika enthalten.
Der Effekt einer Antiangiogenesetherapie kombiniert mit einer Chemotherapie ist von J. Folkman in Nature Medicine 1, 27-30 (1995) beschrieben.

Die Wirksamkeit von Cyclo-(Arg-Gly-Asp-D-Phe-NMe-Val) in Kombination mit einem Chemotherapeutikum kann am Lewis-Lungenkarzinomsystem gezeigt werden. Das Lewis-Lungenkarzinom wird durch konventionelle Chemotherapeutika nur unzureichend beeinflußt (Y. Kakeji und B.A. Teicher, Invest. New Drugs 15: 39-48 (1997)).
Das Verfahren zur Verzögerung des Tumorwachstums wird analog Kakeji durchgeführt (F. Mitjans et al., J. Cell Sci. 108: 2825-2838 (1995)).

Als Chemotherapeutikum kann ein Chemotherapeutikum aus einer Gruppe bestehend aus
a) alkylierenden Agenzien,
b) Antibiotika,
c) Antimetaboliten,
d) Biologika und Immunmodulatoren,
e) Hormonen und deren Antagonisten,
f) Senfgasderivaten,
g) Alkaloiden,
h) Inhibitoren der Matrix-Metallo-Proteinasen (MMP-Inhibitoren),
i) Protein-Kinase-Inhibitoren,
k) anderen
   eingesetzt werden.

Alkylierende Agenzien sind z.B. Busulfan, Carboplatin, Carmustine, Cisplatin, Cyclophosphamid, Dacarbazine, Ifosfamide oder Lomustine. Antibiotika sind z.B. Bleomycin, Doxorubicin (Adriamycin), Idarubicin oder Plicamycin.
Antimetaboliten sind z.B. Sulfonamide oder Folsäureantagonisten, wie z.B. auch 5-Fluorouracil (5-FU), Mercaptopurin, Methotrexat oder Thioguanin oder 5-FU mit Calciumfolinat (Leucovorin).
Biologika und Immunmodulatoren sind z.B. Interferon a2A, Interleukin 2 oder Levamisole.

Hormone und deren Antagonisten sind z.B. Flutamide, Goserelin, Mitotane oder Tamoxifen. Senfgasderivate sind z.B. Melphalan, Carmustine oder Stickstofflost, Alkaloide sind z.B. die Taxane wie Docetaxel oder Paclitaxel, ferner Etoposide, Vinblastine oder Vinovelbine.
Unter anderen Chemotherapeutika werden solche verstanden, die nicht den vorstehenden Gruppen zugeordnet werden können, wie z.B. Altretamine, Cladribine, Gemcitabine, Leucovorin, Levamisole, Pentostatin oder Irinotecan.

Inhibitoren der Matrix-Metallo-Proteinasen (MMP-Inhibitoren), die z.B. auch von M.Wittaker et al. in Current Opinion in Drug Discovery & Development 1, 157-164 (1998*)* beschrieben sind, sind die nachstehenden Verbindungen, z.B. Baltimastat (BB-94), Marimastat (BB-2516), BB-3644, Ilomastat, Metastat, AG-3340, BAY-12-9566, AE-941/Neovastat, CGS-27023A, RS-113456, RS-130830, Ro-32-3555, Ro-31-9790, CT-1746, CT-1418, D-1927, D-2163.

Protein-Kinase-Inhibitoren sind z.B. durch G.McMahon et al. in Current Opinion in Drug Discovery & Development 1, 131-146 (1998*)* und von L.M. Strawn et al. in Exp. Opin. Invest. Drugs 7, 553-573 (1998) beschrieben. Beispiele für Rezeptor-Tyrosin-Kinase-Inhibitoren sind:
CGP 79787, SU-101 (HWA 486, Leflunomide, Arava), SU-5416, SU-5271 (PD-153035), PD-173074, SU-6668, ZD-1839, CP-358774.

Es können auch sogenannte Prodrug-Derivate der Angiogeneseinhibitoren und/oder der Chemotherapeutika verwendet werden, d. h. mit z. B. Alkyl- oder Acylgruppen, Zuckern oder Oligopeptiden abgewandelte Verbindungen, die im Organismus rasch zu den wirksamen erfindungsgemäßen Verbindungen gespalten werden.

Zu den Prodrug-Derivaten gehört z.B. auch das Chemotherapeutikum Capecitabine, welches das Prodrug von 5-FU darstellt, wie dies z. B. in Inpharma No. 1142, 13-14 (1998) beschrieben ist.

Die Verwendung von Gemcitabine bei der Tumorbehandlung ist z.B. von B.J. Braakhuis et al. in Semin-Oncol. 1995 Aug; 22(4 Suppl. 11): 42-6 oder von R.M. Mohammed et al. in Pancreas 1998 Jan; 16 (1): 19-25 beschrieben

Weitere Angiogeneseinhibitoren sind z.B, in Tabelle 1 in WO 9741844 beschrieben.

αᵥβ₃- und αᵥβ₅-Integrininhibitoren sind beispielsweise die in der EP 0 770 622 genannten Verbindungen.

Eine pharmazeutische Zubereitung kann hergestellt werden, indem man Cyclo-(Arg-Gly-Asp-D-Phe-NMe-Val) und/oder eines seiner physiologisch unbedenklichen Salze und gegebenenfalls ein Chemotherapeutikum und/oder eines seiner physiologisch unbedenklichen Salze und gegebenenfalls einen Angiogeneseinhibitor und/oder eines seiner physiologisch unbedenklichen Salze, zusammen mit mindestens einem festen, flüssigen oder halbflüssigen Träger- oder Hilfsstoff in eine geeignete Dosierungsform bringt. Die so erhaltenen Zubereitungen können als Arzneimittel in der Human- oder Veterinärmedizin, insbesondere bei der Behandlung von Tumoren, eingesetzt werden. Als Trägersubstanzen kommen organische oder anorganische Stoffe in Frage, die sich für die enterale (z.B. orale, sublinguale oder rektale), parenterale oder topische (z.B. transdermale) Applikation eignen und mit den Verbindungen nicht reagieren, beispielsweise Wasser, pflanzliche Öle, Benzylalkohole, Polyethylenglykole, Glycerinacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässrige Lösungen, ferner Suspensionen, Emulsionen oder Implantate, für die topische Anwendung Salben, Cremes oder Pflaster. Die Wirkstoffe können auch lyophilisiert und die erhaltenen Lyophilisate z.B. zur Herstellung von Injektionspräparaten verwendet werden. Die Zubereitungen können sterilisiert sein und /oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können auch weitere Wirkstoffe enthalten, z.B. andere blutdrucksenkende oder diuretisch wirkende Stoffe, aber auch Vitamine und/oder Mineralsalze, insbesondere solche, die Stoffwechselvorgänge begünstigen.

Solche Zubereitungen können bei der Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen verwendet. In der Regel werden sie zur Tumorbekämpfung, also zur Inhibierung des Tumorwachstums oder von Tumormetastasen eingesetzt werden.

Verwendet werden kann Cyclo-(Arg-Gly-Asp-D-Phe-NMe-Val) auch in solchen Kombinationspräparaten, die zur Bekämpfung von Krankheiten eingesetzt werden, bei der αᵥ-Integrine, insbesondere αᵥβ₃ und αᵥβ₅, eine Rolle spielen und dabei deren Hemmung Teil der Therapie ist.

Verwendet werden kann Cyclo-(Arg-Gly-Asp-D-Phe-NMe-Val) in der Tumortherapie, auch in Kombination mit einem anderen Angiogeneseinhibitor,
a) bei der chirurgischen Entfernung eines Tumors,
b) bei der Radiotherapie,
c) bei der photodynamischen Therapie,
d) zusammen mit monoklonalen Antikörpern gegen tumorselektive Epitope,
e) zusammen mit Fusionsproteinen,
f) zusammen mit Peptid-Vaccinen und
g) bei der Gentherapie.

Die Dosierungen von Cyclo-(Arg-Gly-Asp-D-Phe-NMe-Val) bzw. dessen Salzen als auch der Chemotherapeutika und/oder der Angiogeneseinhibitoren liegen vorzugsweise zwischen etwa 0,1 und 100 mg, insbesondere zwischen 0,2 und 20 mg, ganz besonders zwischen 0,1 und 10 mg pro Dosierungseinheit. Die tägliche Dosierung liegt vorzugsweise zwischen etwa 0,001 und 1, insbesondere zwischen 0,002 und 0,2 mg/kg Körpergewicht
Während einer Chemotherapie kann das Peptid beispielsweise auch in einer Dosierung von 1-10 mg/kg 2x pro Woche gegeben werden. Die Chemotherapeutika können z.B. auch in einer Dosierung von 1-10 mg/kg einmal pro Woche bis alle 3-4 Wochen verabreicht werden. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung ist daher die Verwendung von Cyclo-(Arg-Gly-Asp-D-Phe-NMe-Val) und/oder einem seiner physiologisch unbedenklichen Salze zur Herstellung eines Arzneimittels zur Bekämpfung von Tumoren bei der Radiotherapie.

Möglich ist ferner die Verwendung der beschriebenen pharmazeutischen Zubereitungen zur Bekämpfung von pathologisch angiogenen Erkrankungen, Thrombosen, Herzinfarkt, koronaren Herzerkrankungen, Arteriosklerose, Tumoren, Osteoporose, Entzündungen und Infektionen.

Möglich ist auch die Verwendung von Cyclo-(Arg-Gly-Asp-D-Phe-NMe-Val) und/oder einem seiner physiologisch unbedenklichen Salze nacheinander oder in physikalischer Kombination mit einem Chemotherapeutikum und/oder einem seiner physiologisch unbedenklichen Salze und/oder einem Angiogeneseinhibitor und/oder einem seiner physiologisch unbedenklichen Salze zur Herstellung eines Arzneimittels zur Bekämpfung von Tumoren.

Möglich ist auch die Verwendung von Cyclo-(Arg-Gly-Asp-D-Phe-NMe-Val) und/oder einem seiner physiologisch unbedenklichen Salze nacheinander oder in physikalischer Kombination mit einem Chemotherapeutikum und/oder einem seiner physiologisch unbedenklichen Salze zur Herstellung eines Arzneimittels zur Bekämpfung von Tumoren.

Möglich ist ferner die Verwendung von Cyclo-(Arg-Gly-Asp-D-Phe-NMe-Val) und/oder einem seiner physiologisch unbedenklichen Salze nacheinander oder in physikalischer Kombination mit einem Chemotherapeutikum und/oder einem seiner physiologisch unbedenklichen Salze zur Bekämpfung von Tumoren.

Die Bestandteile der pharmazeutischen Zubereitung werden vorzugsweise kombiniert verabreicht. Sie können aber auch einzeln gleichzeitig oder aufeinanderfolgend verabreicht werden.

Verwendet werden kann auch ein Set (Kit), bestehend aus getrennten Packungen von
(a) einer wirksamen Menge an Cyclo-(Arg-Gly-Asp-D-Phe-NMe-Val) und
(b) einer wirksamen Menge eines Chemotherapeutikums.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an Cyclo-(Arg-Gly-Asp-D-Phe-NMe-Val) und des Chemotherapeutikums gelöst oder in lyophylisierter Form vorliegt.

Aus der WO 9814192 sind verschiedene biologische Tests bekannt, die geeignet sind, um die Konzentration der Verbindungen zu bestimmen, die einen pharmakologischen Effekt hervorrufen.

Cyclo-(Arg-Gly-Asp-D-Phe-NMe-Val), das Chemotherapeutikum oder der Angiogeneseinhibitor kann, bei Vorliegen einer Säurefunktion, mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann eine Säure mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.

Andererseits kann eine basische Funktion in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Denkbar ist weiterhin eine pharmazeutische Zubereitung enthaltend einen αᵥβ₃- und/oder einen αᵥβ₅-integrininhibitor und/oder eines seiner physiologisch unbedenklichen Salze und mindestens einen MMP-Inhibitor und/oder eines seiner physiologisch unbedenklichen Salze sowie eine pharmazeutische Zubereitung enthaltend einen αᵥβ₃- und/oder einen αᵥβ₅-Integrininhibitor und/oder eines seiner physiologisch unbedenklichen Salze und mindestens einen Tyrosin-Kinase-Inhibitor und/oder eines seiner physiologisch unbedenklichen Salze.

Bevorzugte αᵥβ₃- und αᵥβ₅-integrininhibitoren sind z.B. in der EP 0 770 622, EP 0 710 657, EP 0 820 988, EP 0 820 991, WO 94/12181, WO 94/08577, EP 0 518 586, WO 95/32710, WO 96/00574, WO 96/00730 oder in der DE 198 50 131 beschrieben.

Unter den MMP-Inhibitoren und den Tyrosin-Kinase-Inhibitoren sind die oben genannten bevorzugt.

### Nur zur technischen Information:

### Beispiel zum Testen einerKombinationstherapie;

Verzögerung des Tumorwachstums analog Kakeji (F. Mitjans et al., J. Cell Sci. 108: 2825-2838 (1995)):
Lewis-Lungenkarzinomzellen (2x 10E6) werden in 8-10 Wochen alte C57BL-Mäuse injiziert. Am vierten Tag wird Cyclo-(Arg-Gly-Asp-D-Phe-NMe-Val) (30 mg/kg) täglich i.p. verabreicht. Das Tumorwachstum wird täglich gemessen (B.A. Teicher et al., Int. J. Cancer 57: 920-925 (1994)). Haben die Tumoren ein bestimmtes Volumen von ungefähr 100 mm³ erreicht, beginnen am Tag 7 nach Tumorinoculation verschiedene cytotoxische Kombinationstherapien durch intraperitoneale Injektion. Beispiele: 5-Fluorouracil (30 mg/kg) oder Adriamycin (1,8 mg/kg) werden täglich von Tag 7 bis 11 gegeben. Cyclophosphamid (150 mg/kg), Carmustine (15 mg/kg) oder Gemcitabine (2,5 mg/kg) werden am Tag 7, 9 und 11 gegeben. Cisplatin (10 mg/kg) wird am Tag 7 gegeben.
   Die Tumoren werden dreimal die Woche gemessen bis ein Volumen von ungefähr 500 mm³ erreicht ist. Die Verzögerung des Tumorwachsturns wird als die Zeit berechnet, die ein individueller Tumor benötigt um 500 mm³ zu erreichen im Vergleich zu unbehandelten Kontrollen.
Nur zur technischen Information:
   Die nachfolgenden Beispiele betreffen pharmazeutische Zubereitungen:

### Beispiel A: Injektionsgläser

Eine Lösung von 100 g Cyclo-(Arg-Gly-Asp-D-Phe-NMe-Val) und/oder eines seiner physiologisch unbedenklichen Salze, 100 g des Chemotherapeutikums und 5 g Dinatriumhydrogenphosphat wird in 6 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg der Wirkstoffe.

### Beispiel B: Suppositorien

Man schmilzt ein Gemisch von 20 g Cyclo-(Arg-Gly-Asp-D-Phe-NMe-Val) und/oder eines seiner physiologisch unbedenklichen Salze, 20 g des Chemotherapeutikums mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g Cyclo-(Arg-Gly-Asp-D-Phe-NMe-Val) und/oder eines seiner physiologisch unbedenklichen Salze, 1 g des Chemotherapeutikums, 9,38 g NaH₂PO₄ - 2 H₂O, 28,48 g Na₂HPO₄ - 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg Cyclo-(Arg-Gly-Asp-D-Phe-NMe-Val) und/oder eines seiner physiologisch unbedenklichen Salze, 500 mg des Chemotherapeutikums mit 99,5 g Vaseline unter aseptischen Bedingungen.

### Beispiel E: Tabletten

Ein Gemisch von 1 kg Cyclo-(Arg-Gly-Asp-D-Phe-NMe-Val) und/oder eines seiner physiologisch unbedenklichen Salze, 1 kg des Chemotherapeutikums, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Cyclo-(Arg-Gly-Asp-D-Phe-NMe-Val) und/oder eines seiner physiologisch unbedenklichen Salze, 2 kg des Chemotherapeutikums werden in üblicher Weise in Hartgelatinekapseln gefüllt, so daß jede Kapsel 20 mg des Wirkstoffs enthält.

### Beispiel H: Ampullen

Eine Lösung von 1 kg Cyclo-(Arg-Gly-Asp-D-Phe-NMe-Val) und/oder eines seiner physiologisch unbedenklichen Salze, 1 kg des Chemotherapeutikums in 60 I zweifach destilliertem Wasser wird steril filtriert, in Ampullen abgefüllt, unter sterilen Bedingungen lyophilisiert und steril verschlossen. Jede Ampulle enthält je 10 mg der Wirkstoffe.

### Beispiel I: Set (Kit)

### Zubereitung (Kit) zur parenteralen Anwendung

Die Zubereitung enthält 500 mg Cyclo-(Arg-Gly-Asp-D-Phe-NMe-Val) und/oder eines seiner physiologisch unbedenklichen Salze und 500 mg Gemcitabine Hydrochlorid und wird wie folgt hergestellt:
Je 500 mg der beiden Verbindungen werden in 40 mL destillierten Wassers gelöst. Die Lösungen werden unter sterilen Bedingungen filtriert und in 10 ml Ampullen abgefüllt und lyophilisiert.
Zur intravenösen oder intramuskulären Injektion wird mit 10 mL 5%iger wässriger Dextrose versetzt.

## Patentansprüche

1. Verwendung von Cyclo-(Arg-Gly-Asp-D-Phe-NMe-Val) und/oder einem seiner physiologisch unbedenklichen Salze zur Herstellung eines Arzneimittels zur Bekämpfung von Tumoren bei der Radiotherapie.

## Claims

1. Use of cyclo(Arg-Gly-Asp-D-Phe-NMe-Val) and/or one of its physiologically acceptable salts for the preparation of a medicament for combating tumours in radiotherapy.

## Revendications

1. Utilisation de cyclo(Arg-Gly-Asp-D-Phe-NMe-Val) et/ou de l'un de ses sels physiologiquement acceptables pour la préparation d'un médicament pour combattre les tumeurs pour la radiothérapie.
